# EUROPEAN PATENT APPLICATION

(11) **EP 4 375 374 A2**
(43) Date of publication of application: **29.05.2024**
(21) Application number: 24169862.0
(22) Date of filing: 26.01.2022
(51) Int. Cl.: C12N 15/10

(54) **PURIFICATION OF POLY A-TAGGED PRODUCTS**

(30) Priority: 27.01.2021 GB 202101114
(62) Divisional of application: 22703317.2
(71) Applicant: Cytiva BioProcess R&D AB, 751 84 Uppsala (SE)
(72) Inventor: LUNDBACK, Peter, 751 84 Uppsala (SE); PALMGREN, Ronnie, 751 84 Uppsala (SE); LIND, Ola, 751 84 Uppsala (SE); NORRMAN, Nils, 751 84 Uppsala (SE); CHEEK, Helen, Stevenage, SG1 2FX (GB); BRITO DOS SANTOS, Susana, Stevenage, SG1 2FX (GB); DENKER, Per, 751 84 Uppsala (SE)
(74) Representative: Munter, Ulrika

(57) **Abstract**

The invention relates to a chromatography medium comprising a convection-based chromatography material functionalized with oligo d(T)-ligands, wherein the material comprises polymer nanofibers and is in the form of one or more membrane(s) or sheet(s), wherein the oligo(dT)-ligand is a (d)T₁₀₋₅₀ ligand. The invention also relates to processes for purification of polyA-tagged products, such as mRNA, from a synthetic or biological composition, with the use of the chromatography material.

## Description

### Field of the Invention

The invention relates to processes for purification of poly-tagged products, such as mRNA, from synthetic or biological compositions. The process involves contacting the composition with an oligo d(T)- functionalized chromatography medium comprising a convection-based chromatography material.

### Background

Messenger RNA (mRNA) is the key mediator in the central dogma of molecular biology. Single-stranded mRNA is transcribed from and is complementary to one of the DNA strands of a gene and its protein coding region specify the amino acid sequence of the protein. Prior to its role as a protein encoding template, the mRNA is processed through a series of events that mainly occur in the nucleus either post-transcriptionally or concomitantly with the transcription from the DNA gene template. These critical events include 5'-capping, intron splicing, polyadenylation (poly A) of the 3' end, and shuttling from nucleus to the cytoplasm. All these features serve its own purpose and is critical for overall mRNA stability and modulation of translational efficiency. The 5' cap and the polyA tail are both unique features of mRNA.

Technology advances in the synthetic mRNA field have put synthetic mRNA in the spotlight and is currently evaluated in several preclinical and clinical studies for a variety of diseases.

The production of synthetic mRNA by *in vitro* transcription (IVT) involves the key components 1) DNA template, 2) ribonucleotides and 3) RNA polymerase. The UTR sequences and protein coding sequence are defined by the DNA template. The 3' polyA tail may or may not be designed within the DNA template. If included in the DNA template, the length of the polyA tail is controllable while post-IVT polyA-tailing with a polyA polymerase may be used when not designed within the DNA template. Several different 5'-capping strategies exists, both co- and post-transcriptional approaches.

Oligo(dT) products are commonly used in 'open purifications systems' such as magnetic particles or spin columns. During hybridization (binding phase), the mRNA polyA-tail hybridizes to the Oligo(dT) ligand in high salt buffers. The high conductivity limits electrostatic repulsion of the negatively charged backbones of the polyA and oligo(dT) ligand. This is followed by washing and a mild elution using low conductivity buffers or water which destabilizes the TA pair and allows elution. Undesired contaminants such as proteins, unreacted ribonucleotides, DNA, CAP analogues and partial transcripts that lack the polyA moiety are not retained on the solid support during the hybridization or wash phase.

The length of the Oligo(dT) ligand for the magnetic products usually ranges between 14-30 nucleotides. The small particle sizes provide a large particle surface area per mL and the size of the particles commonly ranges between 1-5 µm. The protocol length for small scale mRNA purification is generally shorter than 1h. Noteworthy, these products were designed and are generally used for purification of an mRNA pool from cell lysates and not for purification of mRNA from an IVT reaction. In addition, the known products are designed to operate in microcentrifuge tubes and given their small size, and consequently low magnetism, it is very unlikely that any of these products are scalable for processing larger sample volumes. In addition, such small particles packed as a chromatography medium would have significantly impaired flow properties as compared to more conventional chromatography resins.

Furthermore, there are several drawbacks using known adsorbent materials for chromatographic mRNA separations.

Separations involving membranes and monoliths can be run at far higher flowrates than porous bead-based systems, typical residence times being in the order of 0.2-0.5 minutes. However, typical binding capacities at 10% breakthrough of target for monoliths and membranes under dynamic flow are lower than porous beads. The inferior binding capacity of monolith and membrane materials (compared to porous bead-based materials) can be offset to some extent by utilising higher flowrates. In (membrane) adsorption chromatography, in contrast to gel-permeation chromatography, there is binding of components of a fluid, for example individual molecules, associates or particles, to the surface of a solid in contact with the fluid without the need for transport in pores by diffusion and the active surface of the solid phase is accessible for molecules by convective transport. The advantage of membrane adsorbers over packed chromatography columns is their suitability for being run with much higher flow rates.

This is also called convection-based chromatography. A convection-based chromatography matrix includes any matrix in which application of a hydraulic pressure difference between the inflow and outflow of the matrix forces perfusion of the matrix, achieving substantially convective transport of the substance(s) into the matrix or out of the matrix, which is effected very rapidly at a high flow rate.

Convection-based chromatography and membrane adsorbers are described in for example US20140296464A1, US20160288089A1, US2019308169A1 and US2019234914A1, hereby incorporated by reference in their entireties.

There exists a need for chromatography materials that can separate mRNA from an *in vitro* transcribed (IVT) reaction to enable a therapeutic product to be recovered at industrial scale. The chromatography materials should also share the high binding capacity that porous bead-based materials have desired molecules and the higher flowrates that are achievable with monolith/membrane materials. The chromatography materials must also be sufficiently porous so the binding area is accessible to the large mRNA and so that suitably high flowrates may be achieved.

### Summary of the Invention

The inventors have found that convection-based chromatography material can be functionalized with oligo d(T) ligands and used to separate polyA-tagged products, such as mRNA, from undesired material. They have found that such materials have high capacities for mRNA at residence times down to 10 seconds. The materials used in the invention provide an optimal pore size distribution giving a high binding capacity for the polyA-tagged product. The porosity is large enough that diffusion is not relevant to obtain maximum binding capacity. It is the interaction between oligo dT and the poly A-tagged product that is decisive for residence time.

In one embodiment, the invention relates to processes for synthetic mRNA purification from an *in vitro* transcribed (IVT) reaction. The process involves contacting the sample with a functionalized chromatography material according to the invention.

In a first aspect, the invention relates to a process for recovering a poly A-tagged product from a composition comprising said product, which process comprises contacting the composition with a chromatography material comprising convection-based chromatography material functionalised with oligo(dT)-ligands.

The oligo(dT)-ligand is preferably a (d)T₁₀₋₅₀ ligand, more preferably a (d)T₁₂₋₃₀ ligand.

According to the invention the oligo(dT)-ligand density on the chromatography material is 10-20 µmole/g. Preferably the oligo(dT)-ligand is coupled to the chromatography material via a C3-C12 linker, such as a C6 or C12 linker.

The chromatography material may comprise one or more non-woven polymer nanofibers, preferably cellulose nanofibers. Alternatively, the chromatography material comprises 3D printed material. Further examples may be found in the detailed section of the invention below.

Preferably the chromatography material is in the form of one or more membrane(s) or sheet(s) and the composition is passed through a holder comprising one or more said membranes or sheets and optionally one or more frits or other spacer materials.

Optionally a heat able metal structure is placed between the membranes or sheets which facilitates the elution of the poly A-tagged product when the device is heated.

According to the invention the composition is contacted with the functionalised chromatography medium for a period of 10-15 seconds.

A process for using the device comprises the steps of:
(i) contacting the composition with the functionalised chromatography medium;
(ii) optionally washing the functionalised chromatography medium with a liquid phase of low ionic concentration; and
(iii) selectively eluting the poly A-tagged product and the product-related impurities by contacting the functionalised chromatography medium with a liquid phase of low/very low ionic strength.

In one embodiment the process comprises the steps of:
(i) contacting a solution comprising the composition with the functionalised chromatography material; and
(ii) collecting the solution that has contacted the functionalised chromatography material in step (i), which solution comprises the poly A-tagged product.

The process may be repeated at least 10 times without cleaning in place (CIP).

In a second aspect the invention relates to a chromatography material comprising a convection-based chromatography material functionalized with oligo d(T)-ligands.

The chromatography material comprises for example polymer nanofibers or a 3D printed structure.

The chromatography material is preferably in the form of one or more membrane(s) or sheet(s). When at least to membranes or sheets are provided, at least one heat able metal structure, such as a metal mesh, optionally may be provided between two membranes or sheets. This structure may be heated during elution of the poly A-tagged product.

### Brief Description of the Figures

Fig 1A is a graph showing the dynamic binding capacity (DBC) for mRNA of different length on a chromatography material of the invention functionalized with oligo d(T) ligands (Fibro); Fig 1B is a table showing the length of the different mRNA's as well as different properties thereof and running parameters; and
Fig 2A is a graph showing the flow rate impact at 10% dynamic binding capacity (DBC) on the same material as in Fig 1A-B (curves representing, from left to right at 10 % DBC, 7.5 s RT, 15 s RT, 30 s RT, 60 s RT, 2 min RT, 4 min RT, 8 min RT and 20 min RT); Fig 2B is a table showing the results for various residence times shown in Fig 2A.
Fig 3 is a graph showing dynamic binding capacity and ligand density for two different ligand lengths, (dT)30 and dT(20), respectively.
Fig 4 is a graph showing showing the impact on dynamic binding capacity of two different linkers, C12 and C6, using the same ligand length as in Fig 3.
Fig 5 is a graph showing the pressure profile, as described in the Example section, for 10 consecutive cycles without cleaning in place (CIP).
Fig 6A is a chromatogram which shows the conductivity and absorbance results of Run 1 as described in the example section. Fig 6B is a chromatogram which shows the conductivity and absorbance results of Run 2 as described in the example section.
Fig 7 shows the breakthrough curve to calculate dynamic binding capacity (DBC) as described in the example section,

### Detailed Description of the Invention

The invention will now be described more closely in relation to some non-limiting Examples and the accompanying drawings.

The chromatography materail according to the present invention comprises convection-based chromatography material. A convection-based chromatography material can be for example an adsorptive membrane where a flow through such materials is convective rather than diffusional. The adsorptive membrane can for example be a polymer nanofiber membrane, such as for example cellulose, cellulose acetate and cellulose fibers which have been treated for use as an adsorbent. The adsorptive membrane could alternatively be a monolithic material or a conventional membrane made by emulsification. Another alternative is a 3D printed material.

Optionally, the adsorptive membrane comprises polymer nanofibers. Typically, the polymer nanofibres are in the form of one or more non-woven sheets, each sheet comprising one or more said polymer nanofibres. A non-woven sheet comprising one or more polymer nanofibres is a mat of said one or more polymer nanofibres with each fibre oriented essentially randomly, i.e. it has not been fabricated so that the fibre or fibres adopts a particular pattern. Non-woven sheets comprising polymer nanofibres are typically provided by known methods. Non-woven sheets may, in certain circumstances, consist of a single polymer nanofibre. Alternatively, non-woven sheets may comprise two or more polymer nanofibres, for example 2, 3, 4, 5, 6, 7, 8, 9 or 10 polymer nanofibres.

The polymer nanofibres may be electrospun polymer nanofibres. Such electrospun polymer nanofibres are well known to the person skilled in the art. Alternative methods for producing polymer nanofibres may also be used, e.g. drawing.

Polymer nanofibres for use in the present invention typically have mean diameters from 10nm to 1000nm. For some applications, polymer nanofibres having mean diameters from 200nm to 800nm are appropriate. Polymer nanofibres having mean diameters from 200nm to 400nm may be appropriate for certain applications.

The length of polymer nanofibres for use in the present invention is not particularly limited. Thus, conventional processes e.g. electrospinning can produce polymer nanofibres many hundreds of metres or even kilometres in length. Typically, though, the one or more polymer nanofibres have a length up to 10km, preferably from 10m to 10km.

Non-woven sheets typically have area densities from 1 to 40g/m², preferably from 5 to 25g/m², in some circumstances from 1 to 20 or 5 to 15 g/m².

Non-woven sheets typically have a thickness from 5 to 120 µm, preferably from 10 to 100 µm, in some circumstances from 50 to 90 µm, in other circumstances from 5 to 40, 10 to 30 or 15 to 25 µm.

The polymer used to produce the nanofibres used in the processes of the present invention is not particularly limited, provided the polymer is suitable for use in chromatography applications. Thus, typically, the polymer is a polymer suitable for use as a chromatography medium, i.e. an adsorbent, in a chromatography method. Suitable polymers include polyamides such as nylon, polyacrylic acid, polymethacrylic acid, polyacrylonitrile, polystyrene, polysulfones e.g. polyethersulfone (PES), polycaprolactone, collagen, chitosan, polyethylene oxide, agarose, agarose acetate, cellulose, cellulose acetate, and combinations thereof. Polyethersulfone (PES), cellulose and cellulose acetate are preferred. In some cases, cellulose and cellulose acetate are preferred.

Typically, the functionalised chromatography material is a functionalised cellulose chromatography material. Preferably, the functionalised chromatography material is formed of one or more non-woven sheets, each comprising one or more cellulose or cellulose acetate nanofibres. Cellulose acetate is readily formed into nanofibres, e.g. by electrospinning and can readily be transformed into cellulose after electrospinning.

Although in a particularly preferred embodiment, the functionalised chromatography material comprises one or more polymer nanofibres, in an alternative embodiment, the functionalised chromatography material may comprise one or more of any type of polymer fibre. Such polymer fibres may have any or all of the same properties as the nanofibres described above. Typically, such polymer fibres may have mean diameters from 10nm to 1000 µm, preferably from 10nm to 750 µm, more preferably from 10nm to 500 µm, even more preferably from 10nm to 400 µm, even more preferably from 10nm to 300 µm, even more preferably from 10nm to 200 µm, even more preferably from 10nm to 100 µm, even more preferably from 10nm to 75 µm, even more preferably from 10nm to 50 µm, even more preferably from 10nm to 40 µm, even more preferably from 10nm to 30 µm, even more preferably from 10nm to 20 µm, even more preferably from 10nm to 10 µm, even more preferably from 10nm to 5 µm, even more preferably from 10nm to 4 µm, even more preferably from 10nm to 3 µm, even more preferably from 10nm to 2 µm, even more preferably from 10nm to 1µm (1000nm).

The nanofibres are functionalised with oligo(dT)-ligand such as a (d)T₁₀₋₅₀ ligand, preferably (d)T₁₂₋₃₀.

Use of multiple non-woven sheets of polymer nanofibres enables a thicker material to be prepared which has a greater capacity for adsorbance. The functionalised chromatography medium is typically therefore formed by providing two or more non-woven sheets stacked one on top of the other, each said sheet comprising one or more polymer nanofibres, and simultaneously heating and pressing the stack of sheets to fuse points of contact between the nanofibres of adjacent sheets.

Preferred processing conditions for pressing and heating of polymer nanofibres/non-woven sheets can be found in WO-A-2015/052460 and WO-A-2015/052465, the entirety of which are incorporated herein by reference.

The functionalised chromatography material has a dynamic binding capacity (DBC) that is dependent of the size of the mRNA and specific examples are given in the examples below. The DBC for 10% breakthrough can be determined in accordance with standard means, e.g. using an AKTA Pure system or equivalent FPLC systems.

DBC for 10% breakthrough is typically determined according to the following assay method:
1) Loading material is passed through functionalised material contained within a holder on an AKTA Pure system (Cytiva);
2) Material is loaded under a determined membrane volume per minute flowrate (mV/min) until the concentration after the holder outlet exceeded 10% of that loaded as determined by the UV flow cell;
3) Accounting for dead volumes in the system and the holder device the total amount of protein loaded onto the disc at the 10% breakthrough was determined through analysis of the chromatogram in the Unicorn software (Cytiva).

The functionalised chromatography material may be housed in a chromatography cartridge or holder. The cartridge typically comprises one or more functionalised chromatography media of the present invention. The cartridge is typically cylindrical.

Typically, the chromatography cartridge comprises one or more functionalised chromatography media of the present invention stacked or wound inside a typically cylindrical holder. The chromatography cartridge may be designed to operate under axial or radial flow.

The processes of the present invention can be operated at high flowrates. Thus, typically in the chromatography process of the present invention, the composition is contacted with the functionalised chromatography material for a period of time of one minute or less, preferably down to 10 seconds.

In mRNA purification the sample is introduced into a column capture chromatography system, such as a functionalized chromatography material used in the present invention, configured for a cyclic purifying process to extract the target product. The cyclic process includes loading the feed onto a unit, washing the unit, eluting the target product and thereafter cleaning the unit before the unit is loaded with new feed. It is desirable to be able to run the unit for several cycles before it needs to be cleaned.

Typically, the process of the invention comprises the steps of:
(i) contacting the composition as defined herein with the functionalised chromatography material as defined herein;
(ii) loading at high ionic strength, optionally washing the functionalised chromatography material with the same buffer as loading and/or lower ionic strength; and
(iii) selectively eluting the mRNA product and the product-related impurities by contacting the functionalised chromatography material with a liquid phase of low/very low ionic strength, such as water.

After the elute step, the process may further comprise a step of regenerating the functionalised chromatography material. Typically this is effected by contacting the functionalised chromatography material from which the mRNA product and/or product related impurities have been eluted with buffer. This can be carried out in accordance with conventional methods known for the regeneration phase of such chromatographic methods.

Typically, the process of recovering a mRNA product in accordance with the present invention comprises a single bind-elute step or a single flow-through step. Alternatively, the process in accordance with the present invention may comprise more than one bind-elute step in series, e.g. two, three, four, five or more bind-elute steps. Alternatively, the process in accordance with the present invention may comprise more than one flow-through step in series, e.g. two, three, four, five or more flow-through steps. Alternatively, the process in accordance with the present invention may comprise a combination of bind-elute and flow-through steps in series, e.g. two, three, four, five or more steps in total.

### EXPERIMENTAL SECTION

### Materials and methods

The data generated and presented in the present invention was performed on a prototype device with oligo(dT)₃₀ ligand or oligo(dT)₂₀ ligand immobilized on a convection-based chromatography material. Prototype A was an oligo (dT)₂₀ ligand with aminated C6 linker immobilized on Fibro VS (vinylsulfone) membrane.

### Synthesis of oligo-dT ligands

All oligo-dT ligands were synthesized using a standard cycle of acid-catalyzed detritylation (3%, v/v, dichloroacetic acid in toluene), coupling (5-(benzylmercapto)-1H-tetrazole (BMT) as activating agent, 0.3 M in acetonitrile), capping (Cap A, 20%, v/v, N-methylimidazole/acetonitrile and an equal volume of B1 (40%, v/v, acetic anhydride in acetonitrile) and B2 (60%, v/v lutidine in acetonitrile) as Cap B were mixed in situ for capping), and iodine-based oxidation (0.05 M iodine in pyridine with 10% v/v water) using 5G UnyLinker polystyrene support on automated solid-phase synthesizer (ÄKTA oligopilot plus 100) and β-cyanoethyl phosphoramidite monomers. The phosphoramidite monomers were dissolved in anhydrous acetonitrile to a concentration of 0.150 M and used in presence of molecular sieves (0.3 nm rods of 1.6 mm). The recycle time used for unmodified phosphoramidites was 3 min (with 1.8 molar equivalence) and amine spacer phosphoramidites were 5 min (with 2.5 molar equivalence). Stepwise coupling efficiencies were found to be >99.0%. After the synthesis of oligo ligands, cleavage from solid support and deprotection of protecting groups were carried out by treating the resin with 25% aq. NH₃ for 12-16 hours at 55 °C. Further, the supernatant solution was collected and the support was washed with water and 50% EtOH in water. The collective fractions were evaporated on a rotary evaporator. The crude ligand pellet was dissolved in water, and the concentration was measured at 260 nm in a UV-VIS spectrophotometer. The purity of ligands was analyzed on IEX-UPLC with tris and sodium perchlorate as running buffer.

Preferably an aminated ligand is used instead of a thiolated ligand due to the preparation of the ligand for immobilisation, although either can be used. A thiolated ligand is provided with the linker as a dimer, which then requires a reduction and desalting step before reacting with the fibres. An aminated ligand is provided with a terminal amine group that is able to react with the fibres without any prior reaction required. There are minor differences in the synthesis of both ligands such as different molar equivalence of thiol or amidite (5-10), recycle times (10-40 min), iodine concentration for oxidation (20-50 mM), oxidation time (2-4 min).

### Preparation of glycidol vinyl sulfone cellulose membrane (Fibro-VS)

50 cellulose acetate disks were washed with distilled water (4x600ml). The wash solution was removed and replaced with 350ml 0.5M KOH solution. The disks were treated with the KOH solution for 10 mins with stirring, before the addition of 100ml glycidol. The reaction media was stirred vigorously over the disks for 2 hours. After this time, the supernatant liquid was removed and the disks washed with distilled water (4X600ml) to give a clean intermediate that was used without further modification for the next step.

Thereafter, 25 disks were taken from the glycidol step and suspended in 500ml H₂O, which contained 37.5g Na₂CO₃ and 150ml MeCN. The mixture was stirred vigorously while 100ml divinyl sulfone was added dropwise over 60 minutes. The reaction mixture was then stirred vigorously for 16 hours. After this time, the supernatant liquid was decanted and the disks washed with 600ml acetone:H₂O (1:1) 3 times and with distilled H₂O (1×600ml). The clean intermediate was used for the next step without further modification.

### Functionalisation of Fibro VS membrane with thiolated oligo dT-ligand

Thiolated oligo dT solution was desalted on an ÄKTA pure with a 50 mL desalting column into 150 mM NaCl. The resulting solution was reduced using 25 mM DTT, 0.1 M NaHCO₃, 0.01M Na₂CO₃ for 1 hour, followed by a further desalting as previously described. The resulting solution was concentrated using 20 mL VivaSpin columns MWCO 5kDa. Solution was then diluted to 5.9 mg/mL, and was added to a Fibro VS sheet in a sealable container before adding sodium sulfate (~3 g). The container was sealed and placed on an orbital shaker for 16 hours. After this time, the supernatant was discarded and DI water (50 mL) was added to each tray. This was repeated 5x in total before any further steps were carried out.

### Functionalisation of Fibro VS membrane with aminated oligo dT-ligand

Aminated oligo dT sample was dissolved in 150mM NaCl buffer (50 mL). Solution was diluted to a concentration of 6.2 mg/mL and a volume of 50 mL by adding DI water (43 mL) to oligo dT solution (7 mL). Sodium sulfate (7.1 g) was added and the pH measured. This solution was added to a T1 sheet of Fibro VS in a sealable container and placed on an orbital shaker for 16 hours. After this time, the supernatant was discarded and DI water (50 mL) was added to the tray before placing back on the orbital shaker. This process was repeated 4x before any further steps were carried out.

### Blocking of divinylsulfone reactive groups

To block any remaining vinylsulfone groups on Fibro VS functionalised with oligo dT, a phosphate buffered solution of thioglycerol (2.5 v/v% thioglycerol, pH 8.3) was prepared by dissolving sodium phosphate dibasic dodecahydrate (3.58 g) and disodium EDTA dihydrate (37 mg) in water (95 mL) with stirring. Thioglycerol (2.5 mL) was added and the resulting solution was basified to pH 8.3 using saturated NaOH solution and diluted to 100 mL.

Sheets of functionalized material were placed in sealable containers and submerged in 25 mL of buffered thioglycerol solution before placing on an orbital shaker for a minimum of 16 h. After this time, thioglycerol solution was discarded and DI water (50 mL) was added to the sheet before placing back on the orbital shaker for a minimum of 15 minutes. This washing process was repeated 3 more times. The final wash was replaced with glycerol:ethanol:water (50 mL, 20:20:60 v/v%) and soaked for 1 hour, then removed wet overmoulded into the desired unit.

### Binding capacity analysis of Fibro Oligo dT20 prototype A

### Materials:

mRNA: Uncapped FlucV01 (2000 nt with 100 nt polyA tail) was produced by *in vitro* transcription and the purified by LiCl precipitation to reach purity of 94% or higher. The mRNA is resuspended in water at around 2mg/ml concentration and stored at -20 degree until being used in chromatography experiments.
Chromatography column: Fibro Oligo dT20 (prototype A) was packed in a PEEK device with 1 layer of membrane, final column volume is 0.2 ml.

### Solutions:

NaCl 5M prepared in RNase-free water
EDTA 500mM, pH 8.0 (RNase-free)
Tris 1M, pH 7.5 (RNase-free)
Binding buffer (inlet A1): NaCl 300 mM, Tris 10 mM, EDTA 1mM pH 7.5
Elution buffer (inlet B 1): RNase-free water
Cleaning-in-place buffer (inlet B2): NaOH 0.1M

### Methods:

### Run 1

For the first run, 5 ml of FlucV01 mRNA at 0.6 mg/mL is prepared by diluting stock mRNA with RNase-free water and then adjust NaCl, Tris and EDTA concentration using stock solution so that the mRNA sample contains NaCl 300 mM, Tris 10 mM, EDTA 1mM pH 7.5. The sample is loaded on a Superloop and is first injected through bypass to measure Amax (or 100% breakthrough) by monitoring UV 260 nm. In order to measure dynamic binding capacity (DBC), mRNA sample is injected onto a PEEK device containing 0.2 ml Fibro Oligo dT20 (Prototype A) while monitoring UV at 260 nm, conductivity, PreColumnPressure and other factors continuously. Flow rate of 2 ml/min was used in all phases, except for during sample application where flow rate was set to 0.4 ml/min to achieve 30 s residence time. After elution, fractions containing mRNA was pooled to calculate recovery percentage.

The following phases were used to bind and elute mRNA.
- Equilibration: 8 ml of binding buffer from inlet A1
- Sample Application: interrupt sample application at 50% of Amax or until the depletion of superloop
- Unbound wash: 2 ml of binding buffer from inlet A1
- Elution: 8 ml of RNase-free water from inlet B 1

The following phases were used to wash the column and prepare it for the next experiment
- Column water wash: 8 ml of RNase-free water from inlet B 1
- CIP: 8 ml of NaOH 0.1M from inlet B2
- Equilibration: 8 ml of binding buffer from inlet A1

DBC is calculated by the below equation:

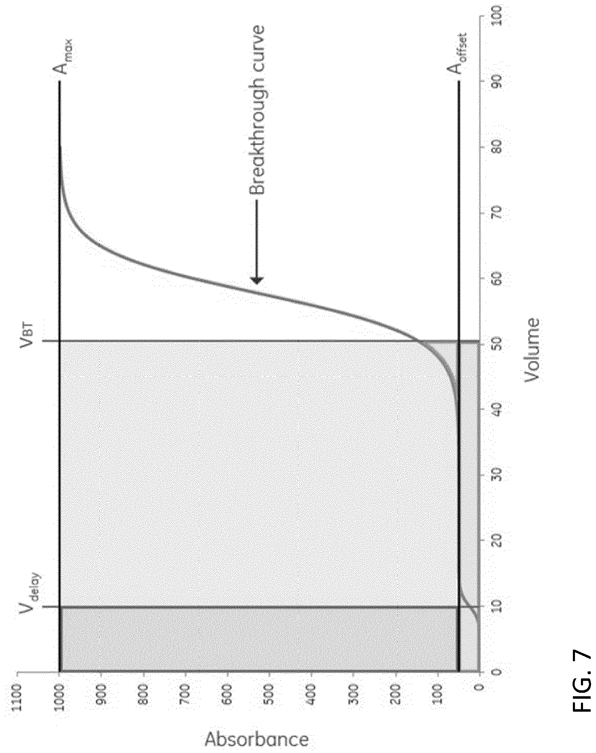

Fig 7 shows the breakthrough curve to calculate the DBC.DBC=Mass of mRNA bound (mg)/Column Volume (ml)=C ×(VBT -Vdelay)/Column Volume (ml) Where C= concentration of mRNA in mg/ml in the feed
VBT= Volume corresponding to the desired breakthrough (ex. 50% in this experiment)
Vdelay= System and column void volume= volume (post injection) at which the conductivity is equal to middle point between the running buffer and the sample input.

### Run 2

For run 2, 14 ml of FlucV01 mRNA at 0.29 mg/mL is prepared by diluting stock mRNA with RNase-free water and then adjust NaCl, Tris and EDTA concentration using stock solution so that the mRNA sample contains NaCl 300 mM, Tris 10 mM, EDTA 1mM pH 7.5. The sample is loaded on a Superloop and is first injected through bypass to measure Amax (or 100% breakthrough) by monitoring UV 260 nm. In order to measure dynamic binding capacity (DBC), mRNA sample is injected onto a PEEK device containing 0.2 ml Fibro Oligo dT20 (batch number 4HC008) while monitoring UV at 260 nm, conductivity, PreColumnPressure and other factors continuously. Flow rate of 2 ml/min was used in all phases, except for during sample application where flow rate was set to 0.4 ml/min to achieve 30 s residence time. After elution, fractions containing mRNA was pooled to calculate recovery percentage.

The following phases were used to bind and elute mRNA.
- Equilibration: 12 ml of binding buffer from inlet A1
- Sample Application: interrupt sample application at 50% of Amax or until the depletion of superloop
- Unbound wash: 4 ml of binding buffer from inlet A1
- Elution: 12 ml of RNase-free water from inlet B 1

The following phases were used to wash the column and prepare it for the next experiment
- Column water wash: 12 ml of RNase-free water from inlet B 1
- CIP: 12 ml of NaOH 0.1M from inlet B2
- Equilibration: 12 ml of binding buffer from inlet A1

Table 1 shows a summary of the running conditions for Run 1 and Run 2.

**Table 1 Running condition for Run 1 and Run 2 - Inlets: A1: NaCl 300 mM, Tris 10 mM, EDTA 1mM pH 7.5 B1: water, B2: NaOH 0.1M, System flow: 2ml/min except for loading.**

| **Method setting** | **Run 1** | **Run2** |
|---|---|---|
| Feed concentration | 0.6 mg/ml | 0.29 mg/ml |
| Feed volume | 5 ml | 14 ml |
| Amax or 100% Breakthrough (mAU) | 2774 | 1440 |
| Equilibration (100% inlet A1) | 8 ml | 12 |
| Sample Application (100% inlet A1 through superloop) | Interrupt application at 1387 mAU or until 7 ml through Superloop | Interrupt application at 720 mAU or until 15 ml through Superloop |
| Unbound wash (100% inlet A1) | 2 ml | 4 ml |
| Elution with water (100% inlet B1) | 8 ml | 12 ml |
| Column water wash (100% inlet B1) | 8 ml | 12 ml |
| CIP with 0.1M NaOH (100% inlet B2) | 8 ml | 16 ml |
| Equilibration (100% inlet A1) | 8 ml | 16 ml |

### Results

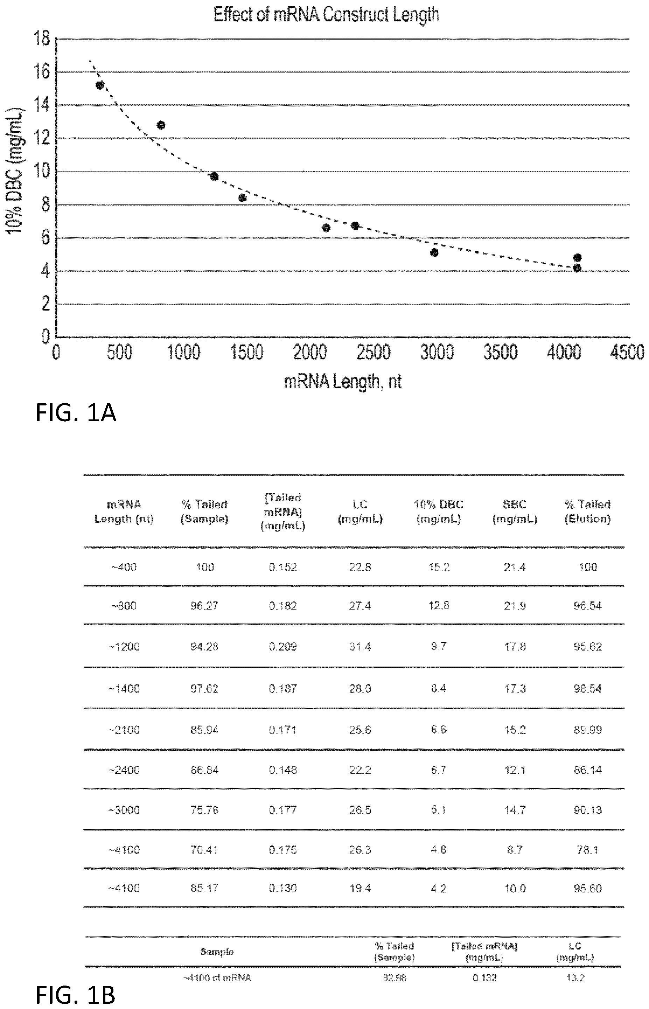

Fig 1A-B show that mRNAs of different length successfully can be purified with a functionalized chromatography medium of the invention. Prototype A was an oligo (dT)₂₀ ligand with aminated C6 linker immobilized on Fibro VS membrane. As appears in the table of the Fig 1B, the mRNA lengths were between about 400 nt to 4100 nt.

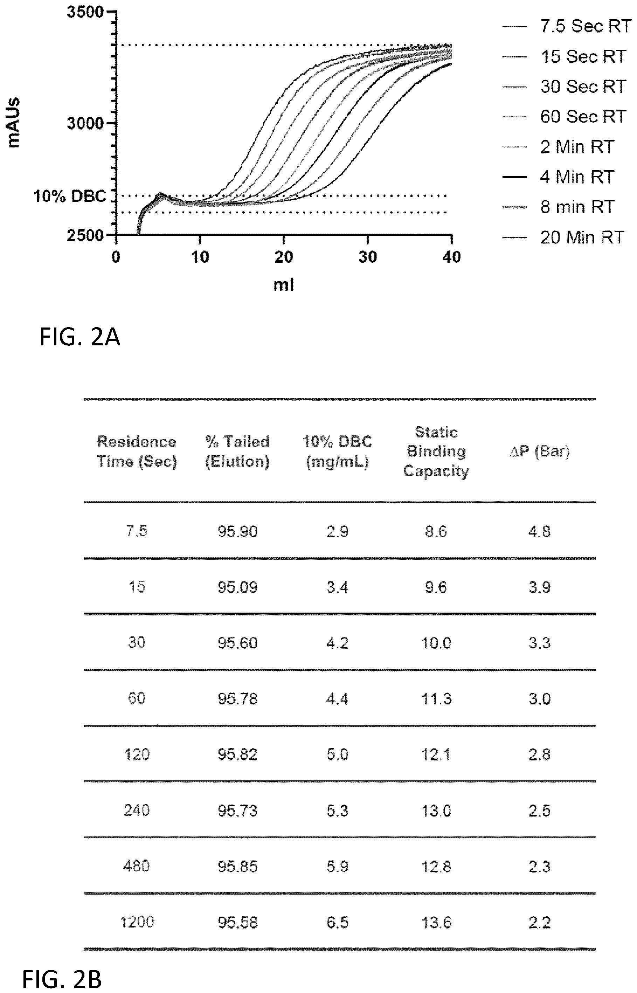

Fig 2A shows the effect of flow rate impact on dynamic binding capacity at different residence times (RT). From left to right in the graph, the curves represent the following residence times: 7.5 sec, 15 sec, 30 sec, 60 sec, 2 in, 4 min, 8 min and 20 min, respectively. Elution data, 10% DBC (mg/ml), static binding capacity and pressure is presented in Fig 2B.

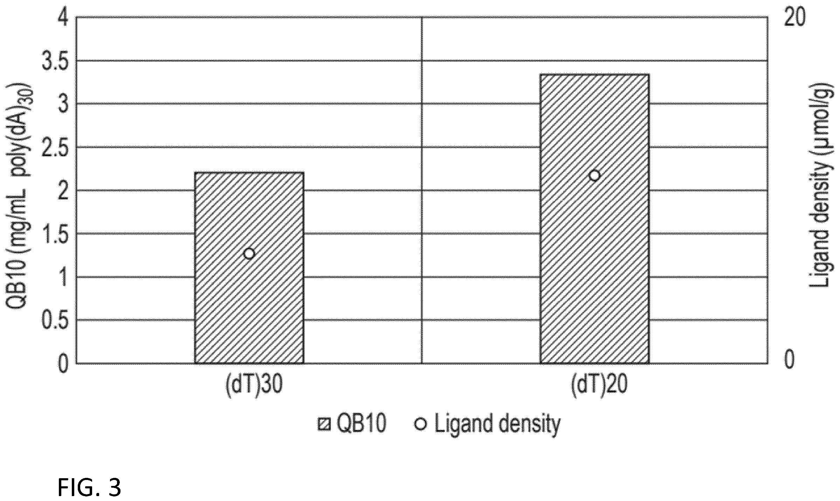

Figure 3 shows the effect of length of the oligo(dT) ligand on dynamic binding capacity and ligand density. Dynamic binding capacity was determined by loading poly(dA)₃₀ oligonucleotide (mRNA surrogate), diluted in binding buffer, until 10% breakthrough (24 seconds residence time). Binding buffer is composed by 10 mM Tris, 400mM NaCl, 1mM EDTA, pH 7.4. Ligand density was determined by Phosphor ICP-SFMS.

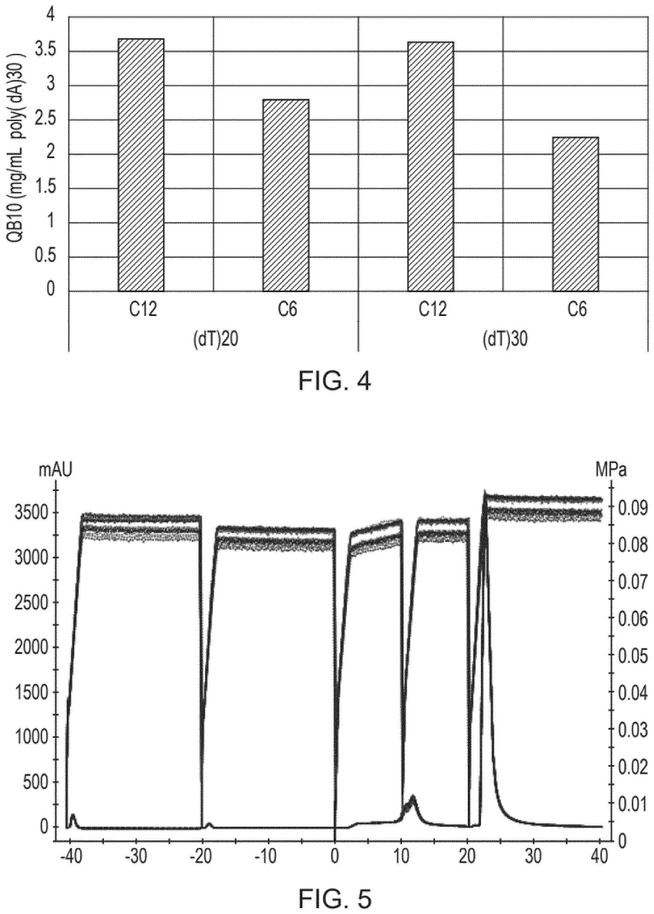

Figure 4 shows the effect of ligand linker on dynamic binding capacity. Dynamic binding capacity was determined as described for Figure 3.

Figure 5 shows the consistent pressure profile over 10 consecutive cycles without CIP. These runs were performed using a poly(dA)₃₀ oligonucleotide as mRNA surrogate, as described in Figure 3

The excellent flow properties of the prototype are preferably utilized in larger devices. A 50 mL device would likely provide acceptable capacity and with a reduced loading time for a 1 L feed corresponding to 20 min as compared to 2500 minutes for a 0.4 mL device.

### Dynamic binding capacity (DBC) experiments

In two independent experiments, the dynamic binding capacity of Fibro Oligo dT20 (Prototype A) was tested in ÄKTA pure chromatography system using in-house produced mRNA.

### Run 1

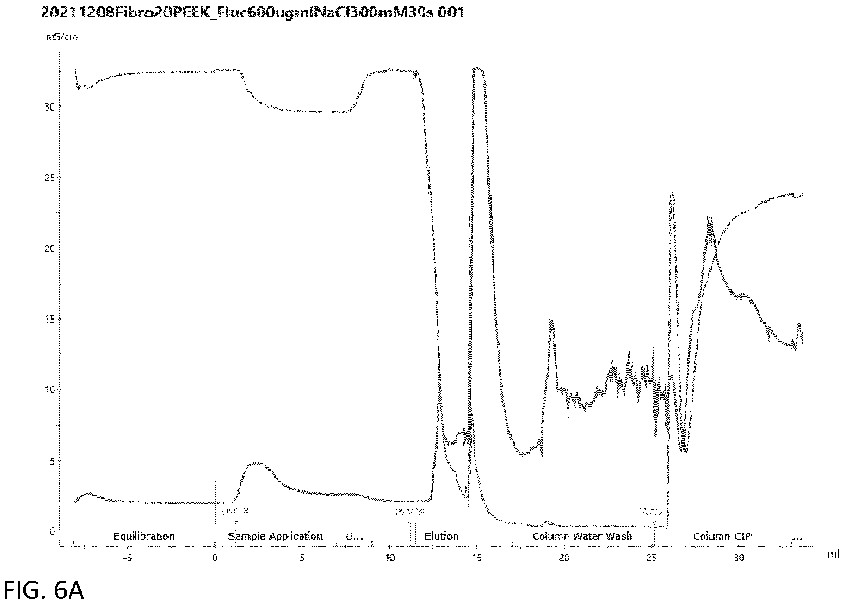

Fig 6A shows the chromatogram for run 1.

Since no breakthrough was detected until the sample is depleted for this run, DBC can only be estimated as larger than the current bound mass DBC*.

DBC > DBC*= Mass of mRNA bound (mg)/ Column Volume (ml)= 0.6 mg/ml*5 ml/0.2 ml=15 mg/ml.

### Run 2

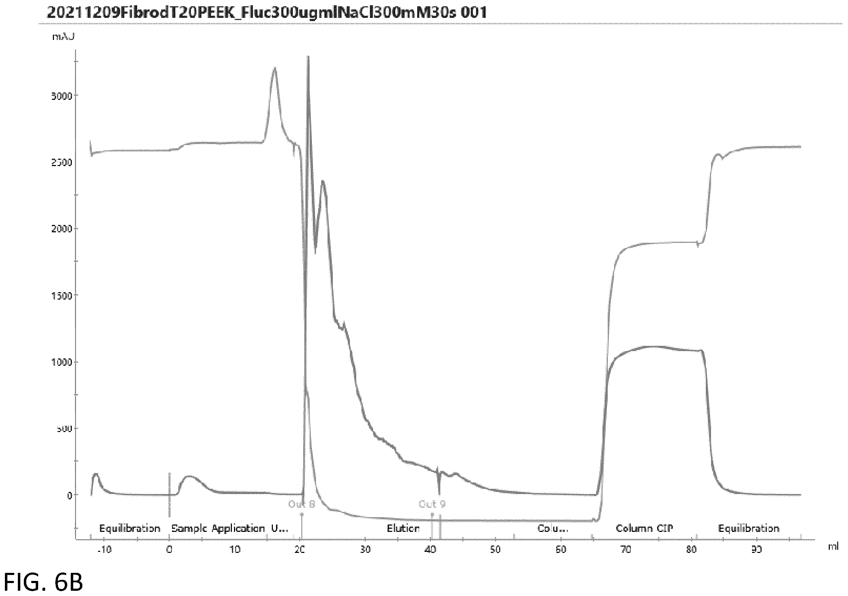

Fig 6B shows the chromatogram for run 2.

Although more mRNA is loaded during run 2, breakthrough was still not detected until the sample is depleted, DBC can only be estimated as larger than the current bound mass DBC*. DBC > DBC*= Mass of mRNA bound (mg)/ Column Volume (ml)= 0.29 mg/ml* 14 ml/0.2 ml=20.3 mg/ml

These two experiments show the excellent properties of Fibro oligo dT compared to other Oligo dT formats that are available on the market. The DBC are in the range of 15 to 20 mg/ml for m-RNA with 2000bp with a residence time under 1 min and a yield above 85 %, the results are summarized in Table 2.

The linker used was C6 aminated, if a C12 aminated linker is used the DBC could be improved further which the data in Fig 7 indicates.

**Table 2: Results Summary for Oligo dT20 C6 runs in Peek device with m-RNA (2000 bp)**

| Fibro Oligo dT in PEEK (dT20 C6) | Run1 | Run2 |
|---|---|---|
| Buffer | Binding: NaCl 300 mM, Tris 10 mM, EDTA 1mM, pH 7.5. | |
| | Elution: water | |
| Elution peak width | Majority within 12 ml | Majority within 10 ml |
| Feed concentration | 0.6 mg/ml | 0.29 mg/ml |
| Feed volume | 5 ml | 14 ml |
| Amax or 100 % Breakthrough (mAU) | 2774 | 1440 |
| Residence time during sample application | 30 still 5.4 then increased to 70 s (flow drop from 0.4 to 0.17 ml/min) | 30 still 7.7 ml then increased to 92 s (flow drop from 0.4 to 0.13 ml/min) |
| DBC (mg mRNA/ml fibro) | > 15 mg/ml | > 20 mg/ml |
| Recovery in elution | 93% | 86 % |
| Pressure plateau at equivalent DBC (mg/ml) | 1.8 MPa @5.4 ml | 1.8MPa @7.7 ml |
| | eqv DBC: 16.2 mg/ml | eqv DBC: 11.2 mg/ml |
| CIP | 0.1M NaOH for 160 CV (8 ml) | 0.1M NaOH for 320 CV (16 ml) |

### Itemized embodiments

1. A process for recovering a poly A-tagged product from a composition comprising said product, which process comprises contacting the composition with a chromatography material comprising convection-based chromatography material functionalised with oligo(dT)-ligands, wherein the oligo(dT)-ligand is a (d)T₁₀₋₅₀ ligand, preferably a (d)T₁₂₋₃₀ ligand.
2. Process according to item 1, wherein the poly A-tagged product is mRNA or any polymer that carries genetic information to ribosome for translation into an amino acid sequence, i.e. peptide or protein.
3. Process according to item 1 or 2, wherein the oligo(dT)-ligand density on the chromatography material is 10-20 µmole/g.
4. Process according to one or more of the above items, wherein the oligo(dT)-ligand is coupled to the chromatography material via a linker, such as a C3-C12 linker.
5. Process according to one or more of the above items, wherein the oligo(dT)-ligand is thiolated or aminated.
6. Process according to one or more of the above items, wherein the chromatography material comprises one or more non-woven polymer nanofibers, preferably cellulose nanofibers.
7. Process according to any one of the preceding items, wherein the chromatography material is in the form of one or more membrane(s) or sheet(s).
8. Process according to any one of the preceding items, wherein the chromatography material is in the form of a membrane or sheet and the composition is passed through a holder or column comprising one or more said membranes or sheets and optionally one or more frits or other spacer materials.
9. Process according to item 8, wherein a heatable metal structure is placed between the membranes or sheets.
10. Process according to any of the preceding items, wherein the composition is contacted with the functionalised chromatography material for a period of 1 minute or less, such as down to 10 seconds.
11. Process according to any of the preceding items, comprising the steps of:
   (i) contacting the composition with the functionalised chromatography material;
   (ii) optionally washing the functionalised chromatography material with a liquid phase of low ionic concentration; and
   (iii) selectively eluting the poly-A tagged product and the product-related impurities by contacting the functionalised chromatography medium with a liquid phase of low/very low ionic strength.
12. A process according to any of items 1 to 10, comprising the steps of:
   (i) contacting a solution comprising the composition with the functionalised chromatography material; and
   (ii) collecting the solution that has contacted the functionalised chromatography material in step (i), which solution comprises the polyA-tagged product.
13. The process according to item 11 or 12, wherein the process is repeated at least 10 times without cleaning in place (CIP).
14. A chromatography material comprising a convection-based chromatography material functionalized with oligo d(T)-ligands, wherein the material comprises polymer nanofibers and is in the form of one or more membrane(s) or sheet(s), wherein the oligo(dT)-ligand is a (d)T₁₀₋₅₀ ligand, preferably a (d)T₁₂₋₃₀ ligand.
15. Chromatography medium according to item 14, comprising a C3-C12 linker between the ligand and the convection-based material.
16. Chromatography medium according to item 15, wherein the linker is a C12 linker and the oligo(dT) ligand is an oligo d(T)₂₀ ligand.
17. Chromatography medium according to item 14, 15 or 16, wherein at least two membranes or sheets are provided and at least one heatable metal structure is provided between two membranes or sheets.

## Claims

1. A chromatography medium comprising a convection-based chromatography material functionalized with oligo d(T)-ligands, wherein the material comprises polymer nanofibers and is in the form of one or more membrane(s) or sheet(s), wherein the oligo(dT)-ligand is a (d)T₁₀₋₅₀ ligand.

2. Chromatography medium according to claim 1, wherein the oligo(dT)-ligand is a (d)T₁₂₋₃₀ ligand.

3. Chromatography medium according to claim 1 or 2, comprising a C3-C12 linker between the ligand and the convection-based material.

4. Chromatography medium according to claim 3, wherein the linker is a C12 linker.

5. Chromatography medium according to any one of the preceding claims, wherein the oligo(dT)-ligand density on the chromatography material is 10-20 µmole/g.

6. Chromatography medium according to any one of the preceding claims, wherein the oligo(dT)-ligand is thiolated or aminated.

7. Chromatography medium according to any one of the preceding claims, comprising wherein the membrane(s) or sheet(s) comprises non-woven polymer nanofibers, preferably cellulose nanofibers.

8. Chromatography medium according to any one of the preceding claims, said one or more membrane(s) or sheet(s) are provided in a holder comprising one or more frits or other spacer materials.

9. Chromatography medium according to any one of the preceding claims, wherein at least one heatable metal structure is provided between two membranes or sheets.

10. A process for recovering a poly A-tagged product from a composition comprising said product, which process comprises contacting the composition with a chromatography medium according to any one of the claims 1-9.

11. Process according to claim 10, wherein the poly A-tagged product is mRNA or any polymer that carries genetic information to ribosome for translation into an amino acid sequence, i.e. peptide or protein.

12. Process according to claim 10 or 11, wherein the composition is contacted with the chromatography material for a period of from 10 seconds to 1 minute.

13. Process according to any one of claims 9-11, comprising the steps of:
(i) contacting the composition with the functionalised chromatography material;
(ii) optionally washing the functionalised chromatography material with a liquid phase of low ionic concentration; and
(iii) selectively eluting the poly-A tagged product and the product-related impurities by contacting the functionalised chromatography medium with a liquid phase of low/very low ionic strength.

14. A process according to any one of claims 10-13, comprising the steps of:
(i) contacting a solution comprising the composition with the functionalised chromatography material; and
(ii) collecting the solution that has contacted the functionalised chromatography material in step (i), which solution comprises the polyA-tagged product.

15. The process according to claim 13 or 14, wherein the process is repeated at least 10 times without cleaning in place (CIP).
